(19) Europäisches Patentamt
European Patent Office
Office européen des brevets

(11) Veröffentlichungsnummer: **0 211 102**
B1

(12) # EUROPÄISCHE PATENTSCHRIFT

(45) Veröffentlichungstag der Patentschrift:
**14.11.90**

(51) Int. Cl.⁵: **C07C 229/44,** A61K 31/215

(21) Anmeldenummer: **85109991.1**

(22) Anmeldetag: **08.08.85**

(54) **Ester der 0(2,6-Dichlorphenylamino)phenylessigsäure und ein Verfahren zur deren Herstellung.**

(43) Veröffentlichungstag der Anmeldung:
**25.02.87 Patentblatt 87/9**

(45) Bekanntmachung des Hinweises auf die Patenterteilung:
**14.11.90 Patentblatt 90/46**

(84) Benannte Vertragsstaaten:
**AT CH DE FR GB IT LI SE**

(56) Entgegenhaltungen:
**EP-A- 0 112 130**
**DE-A- 3 407 507**

**PATENT ABSTRACTS OF JAPAN, Band 9, Nr. 5 (C-260)[1728], 10. Januar 1985; & JP - A - 59 161 330 (ESUESU SEIYAKU) 12.09.1984, & CHEMICAL ABSTRACTS, Band 102, Nr. 13, 1. April 1985, Seite 671, Spalte 1, Zusammenfassungsnr. 113060n**

(73) Patentinhaber: **T P O "PHARMACHIM", Iliensko Chaussée 16, Sofia(BG)**

(72) Erfinder: **Sabunova, Orhideja Bontsheva, Komplex Mladost-2 Block 215-D, Sofia(BG)**
Erfinder: **Zvetanov, Hristo Borissov, Komplex Mladost-2 Block 215-D, Sofia(BG)**
Erfinder: **Petrov, Ljudmila Vutova, 99, Boul. Tolbuhin, Sofia(BG)**
Erfinder: **Ognjanova, Vaska Alexandrova, 12, Aleko Konstantinov Str., Sofia(BG)**
Erfinder: **Nikolova, Milka Petrova, 3, Raiko Daskalov Str., Sofia(BG)**
Erfinder: **Tanev, Georgi Ljubenov, Boul. Vitoscha Nr. 86, Sofia(BG)**
Erfinder: **Ivanova, Nedjalka Stojanova, Dr., 18, Traitcho Kostov Str., Sofia(BG)**

(74) Vertreter: **von Füner, Alexander, Dr. et al, Patentanwälte v. Füner, Ebbinghaus, Finck Mariahilfplatz 2 & 3, D-8000 München 90(DE)**

EP 0 211 102 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft biologisch aktive Ester der 0(2,6-Dichlorphenylamino)phenylessigsäure und ein Verfahren zu deren Herstellung.

Es ist ein Natriumsalz der 0(2,6-Dichlorphenylamino)phenylessigsäure (das Präparat Feloran) bekannt, das antiseptische (entzündungshemmende) und antirheumatische Wirkung besitzt, jedoch die Magenschleimhaut reizt (1. Clinical Pharmacology and Therapeutics St. Louis, 26, (3), 399-405, 1979, 2. Winter C.A., E.A. Risley, G.W. Nuss, Proc. Soc. Exp. Biol. Med., 111, 544-547, 1962, 3. Parrat J.R., G.E. West, J. Physiol., London, 139, 27-41, 1957, 4. Domenjcz R., Actualités Pharmacol., Paris, 7, 8-12, 1954).

Es ist auch ein 2-[(2,6-Dichlorophenyl)amino]-phenylessigsäure-l'-ethoxycarbonyloxyethylester bekannt, der entzündungshemmende Wirkung besitzt (EP-A-0 112 130, TEVA Phar. Indus.)

Aufgabe der Erfindung war es nun, neue Ester der 0(2,6-Dichlorphenylamino)phenylessigsäure zu synthetisieren, welche eine gute entzündungshemmende Wirkung und gleichzeitig keine die Magenschleimhaut reizende Wirkung besitzen sowie auch ein Verfahren zu deren Herstellung zu erarbeiten.

Die erfindungsgemäßen Verbindungen stellen Ester der 0(2,6-Dichlorphenylamino)phenylessigsäure mit Polyäthylenglykol dar und weisen die allgemeine Formel auf

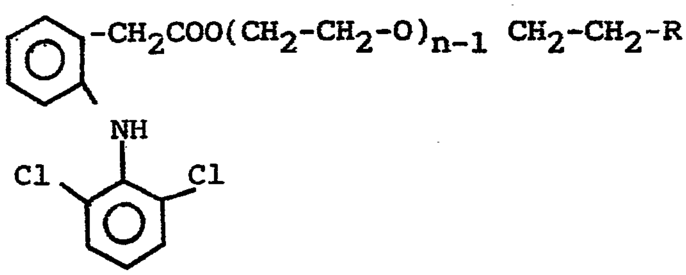

(Ia,b)

wobei bei Ia R

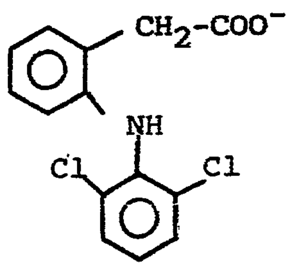

bei Ib R -OH bedeutet und n die Werte 10-450 annimmt mit einer nach dem Dampfdruck-Verfahren bestimmten Molekularmasse von 700 bis 30000.

Das erfindungsgemäße Verfahren zur Herstellung der Verbindungen der allgemeinen Formel I-a, b besteht in der Umsetzung der 0(2,6-Dichlorphenylamino)phenylessigsäure der allgemeinen Formel:

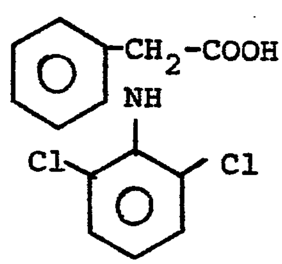

mit Polyäthylenglykol der allgemeinen Formel:

$HO(CH_2\,CH_2O)_nH$,

in welcher n Werte von 10 bis 450 annimmt, in Anwesenheit katalytischer Säuremengen, wie zum Beispiel Chlorosulphon-, p-Toluolsulphon-Säure, in einem inerten Lösungsmittel, z.B. aromatischen Kohlenwasserstoffen (Benzol, Toluol) oder chlorierten Kohlenwasserstoffen (Tetrachlormethan), mittels Erhitzen

unter azeotroper Ausscheidung von Wasser oder in Anwesenheit eines Molekularsiebs. Nach Reaktionsschluß und Neutralisieren der Lösung wird das Produkt durch Umfällen mit Diäthyläther isoliert.

Die Vorteile der erfindungsgemäßen Ester bestehen darin, daß diese viel schwächer die Magenschleimhaut reizen, wobei der entzündungshemmende Effekt mit dem des Felorans gleich ist.

Anhand der nachstehend angeführten Beispiele wird die Herstellung der neuen Ester der 0(2,6-Dichlorphenylamino)phenylessigsäure mit Polyäthylenglykol näher erläutert.

Beispiel 1

1,48 g (0,005 Mol) 0(2,6-Dichlorphenylamino)phenylessigsäure wird in 20 ml Benzol suspendiert, wonach 3,37 g (0,0025 Mol) Polyäthylenglykol mit einer durchschnittlichen Molmasse von 1350, in 20 ml Benzol gelöst, und 0,5 g p-Toluolsulphonsäure zugefügt werden. Die Lösung wird umgerührt und bei 60°C während 10 Stunden in Anwesenheit eines Molekularsiebs erhitzt. nach Reaktionsschluß wird das Benzol unter Vakuum abdestilliert. Dann werden 30 ml Chloroform zugegeben und die Chloroformlösung mit 0,1 N NaOH-Lösung (2,25 ml) und mit Wasser (2,25 ml) gewaschen, mit $NA_2SO_4$) getrocknet, wonach das Chloroform unter Vakuum abdestilliert wird. Das Produckt wird mit Benzol-Diäthyläther (1:1) gereinigt. Es wird zum Kristallisieren bei 10°C gestellt, wonach es filtriert und mit Diäthyläther gespült wird.

Die Ausbeute beträgt 4,3 g oder 90% der Theorie für die Verbindung Ia. Das infrarote Spektrum weist ein spezifisches Band $\nu_{-COO}$-1720 $cm^{-1}$ auf. Das ultraviolette Spektrum in Äthanol - $\lambda_{max}$ ist 280 nm. Die durchschnittliche numerische Molmasse, bestimmt nach dem Dampfdruck-Verfahren beträgt 1900.

Die Verbindung der Formel Ib, in der R OH bedeutet, wird auf analoge Weise hergestellt, wobei das Polyäthylenglykolquantum mit durchschnittlicher Molekularmasse von 1350 6,75 g (0,005 Mol) beträgt. Die Ausbeute der Verbindung Ib beläuft sich auf 7,3 g oder 88% der Theorie. Das infrarote Spektrum weist ein charakteristisches Band $\nu_{-COO}$1720 $cm^{-1}$ und $\nu_{-OH}$3500$cm^{-1}$ auf. Das ultraviolette Spektrum in Äthanol -$\lambda_{max}$ist 280 nm. Die durchschnittliche numerische Molmasse beträgt 1600.

Beispiel 2

Bei der Herstellung der Verbindung Ia mit einer Mol-Masse von 10600 und Verbindung Ib mit Molmasse von 10300 wird wie in Beispiel 1 verfahren, wobei das Polyäthylenglykolquantum mit einer durchschnittlichen Molmasse von 10000 bei der Verbindung Ia 25 g und die Ausbeute 80 % der Theorie beträgt; die Verbindung Ib - 50 g und die Ausbeute ist ebenso 80 % der Theorie. Das infrarote Spektrum der Ia-Verbindung weist $\nu_{-COO}$1720 $cm^{-1}$ auf, und bei der Verbindung Ib ist es $\nu_{-COO}$1720 $cm^{-1}$ und $\nu_{-OH}$3500 $cm^{-1}$. Das ultraviolette Spektrum der Verbindungen Ia und Ib in Äthanol -$\lambda_{max}$ist 280 nm.

Der Diester der 0(2,6-Dichlorphenylamino)phenylessigsäure mit Polyäthylenglykol mit einer durchschnittlichen Molmasse von 1350 (Verbindung Ia) wurde als entzündungshemmendes Mittel bei peroraler Verabreichung geprüft und seine Wirkung mit der antiseptischen Wirkung des bekannten Präparats Feloran verglichen. Ebenso wurde ihre Reizbarkeit auf die Magenschleimhaut verglichen.

An weißen Ratten der Vistar-Rasse wurden Entzündungsmodelle mit Karagenin nach dem Verfahren von Winter et al (Winter C.A., E.A. Risley, G.W. Nuss, Proc. Soc. Exp. Biol.Med., 111, 544-547, 1962), mit Dextran nach dem Verfahren von Parrat und West (Parrat J.R., G.E. West, J. Physiol., London, 139, 27-41, 1957) geschaffen und mit Formalin nach dem Domenjcz R.-Verfahren (Domenjcz R., Actualités Pharmacol., Paris, 7, 8-12, 1954). Die Rattenanzahl pro Dosis beträgt je 10. Bei den drei Entzündungsmodellen mit Dosen 2,5 und 3 mg/kg weist der Diester dieselbe entzündungshemmende Aktivität im Vergleich zu Feloran (Kontrollprobe) auf.

An weißen Ratten der Vistar-Rasse mit je 6 Stück pro Gruppe wurde die Reizbarkeit des Diesters der 0(2,6-Dichlorphenylamino)phenylessigsäure mit Polyäthylenglykol mit durchschnittlicher Molmasse von 1350 (Verbindung Ia) auf die Magenschleimhaut, verglichen mit der Reizbarkeit des Präparats Feloran in Dosen 8 und 10 mg/kg geprüft. Die Befunde zeigen, daß bei Dosen von 8 mg/kg der Diester bei einem Tier Magenschleimhautblutungen gezeigt hat, bei Feloran alle sechs Tiere makroskopische Geschwüre hatten. Bei einer Dosis von 10 mg/kg hatten 3 von den Tieren, mit Diester behandelt, kleine Geschwüre, während bei Feloran alle sechs Tiere große Geschwüre zeigten.

Von den pharmakologischen Vergleichsversuchen des Diesters der 0(2,6-Dichlorphenylamino)phenylessigsäure mit Polyäthylenglykol mit einer durchschnittlichen Molmasse von 1350 und Feloran bei peroraler Verabreichung an Ratten ist der identische entzündungshemmende Effekt festzustellen. Der Diester weist ums doppelte schwächere Reizbarkeit auf die Magenschleimhaut auf, demzufolge er dem Feloran vorzuziehen ist.

## Patentansprüche

1. Ester der 0(2,6-Dichlorphenylamino)phenylessigsäure der allgemeinen Formel

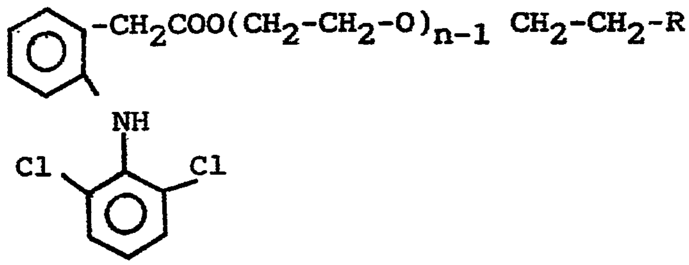

(Ia,b)

worin bei Ia R

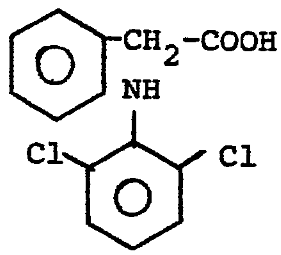

bei Ib R - OH bedeutet und n Werte von 10 bis 450 annimmt mit einer Molmasse von 700 bis 30000, bestimmt nach dem Dampfdruck-Verfahren.

2. Verfahren zur Herstellung von Ester gemäß Patentanspruch 1, dadurch **gekennzeichnet,** daß man 0(2,6-Dichlorphenylamino)phenylessigsäure mit Polyäthylenglykol der allgemeinen Formel HO(CH-CHO)$_n$H, in der n die Werte 10-450 annimmt, in Anwesenheit von Säure in einem Medium von inertem Lösungsmittel unter Erhitzen umsetzt.

3. Arzneimittel, dadurch **gekennzeichnet,** daß es Ester der 0(2,6-Dichlorphenylamino)phenylessigsäure der in Anspruch 1 angegebenen Formel und gegebenenfalls übliche Zusatzstoffe enthält.

## Claims

1. The o(2,6-dichlorophenylamino)phenyl acetic acid ester of the general formula

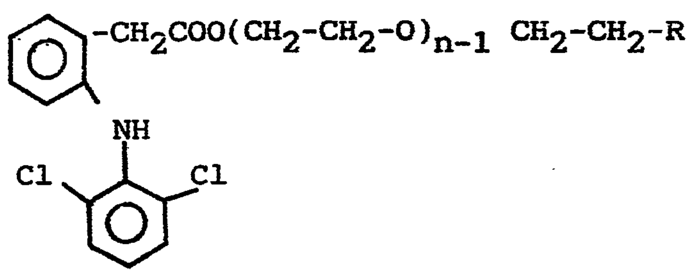

(Ia,b)

wherein, in Ia, R denotes

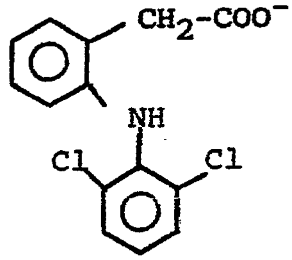

and wherein in Ib R denotes OH, and n assumes values of 10 to 450, with a molar mass of 700 to 30,000 determined by the vapour pressure process.

2. Process for the production of esters according to claim 1, characterised in that o(2,6-dichlorophenylamino)phenyl acetic acid is reacted with polyethylene glycol of the general formula $HO(CH–CHO)_nH$, in which n assumes the value of 10–450, in the presence of acid in an inert solvent medium, while undergoing heating.

3. Medicament, characterised in that it contains the o(2,6-dichlorophenylamino)phenyl acetic acid ester of the formula indicated in claim 1, and optionally conventional additives.

**Revendications**

1. Ester de l'acide o-(dichloro-2,6 phénylamino)phénylacétique, représenté par la formule générale:

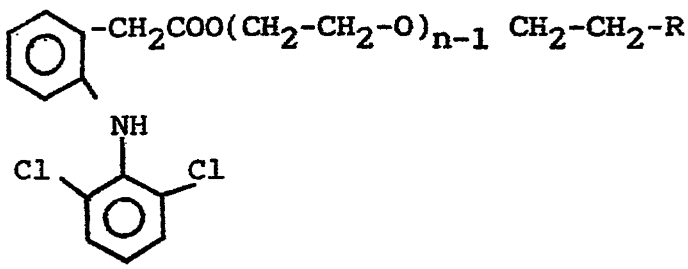

(Ia,b)

où:

dans le cas de (IA), R signifie

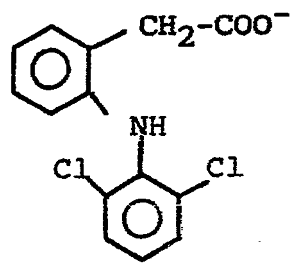

dans le cas de (Ib), R signifie OH, et

n prend des valeurs de 10 à 450, avec une masse moléculaire de 700 à 30 000, définie selon le procédé de tension de vapeur.

2. Procédé de fabrication de l'ester selon la revendication 1, caractérisé par le fait qu'on fait réagir de l'acide o-(dichloro-2,6 phénylamino)phénylacétique avec du polyéthylène glycol de formule générale $HO(CH–CHO)_nH$, dans laquelle n prend les valeurs de 10–450, en présence d'acide dans un milieu de solvant inerte, sous chauffage.

3. Médicament caractérisé par le fait qu'il contient un ester de l'acide o-(dichloro-2,6 phénylamino)-phénylacétique de la formule indiquée à la revendication 1 et, le cas échéant, des adjuvants usuels.